Europäisches Patentamt

European Patent Office

Office européen des brevets

(19)

(11) Publication number: **0 023 196**
**B1**

## (12) EUROPEAN PATENT SPECIFICATION

(45) Date of publication of patent specification: **21.09.83**

(51) Int. Cl.³: **A 61 L 2/06**

(21) Application number: **80850103.5**

(22) Date of filing: **25.06.80**

(54) **Method of disinfecting objects used for the personal care of patients and arrangement in a flusher disinfector for the purpose.**

(30) Priority: **12.07.79 SE 7906061**

(43) Date of publication of application:
**28.01.81 Bulletin 81/4**

(45) Publication of the grant of the patent:
**21.09.83 Bulletin 83/38**

(84) Designated Contracting States:
**AT BE CH DE GB LI NL**

(56) References cited:
**DE - A - 2 415 963**
**FR - A - 1 565 407**
**SE - A - 321 547**

(73) Proprietor: **AKTIEBOLAGET ELECTROLUX**
**Luxbacken 1**
**S-105 45 Stockholm (SE)**

(72) Inventor: **Häkansson, Paul Johan Reinhold**
**Diskusvägen 5**
**S-352 51 Växjo (SE)**
Inventor: **Bergqvist, Jan Ake**
**Bergundavägen 18**
**S-352 35 Växjö (SE)**

(74) Representative: **Hagelbäck, Evert Isidor et al,**
**c/o AB Electrolux Patentavdelningen**
**S-105 45 Stockholm (SE)**

Courier Press, Leamington Spa, England

Method of disinfecting objects used for the personal care of patients
and arrangement in a flusher disinfector for the purpose

The present invention relates to a method of disinfecting objects used for the personal care of patients and to an arrangement in a flusher disinfector with a chamber for the goods and a steam generator for performing the method.

It is known to use a flusher disinfector in a disinfecting room connecting on a hospital ward or a nursing home. The apparatus is primarily intended for emptying, cleaning and disinfecting recirculate equipment for the personal care of the patients. Such items are for example buckets and urine bottles or wash basins, bidets and kidney basins etc. In the first case the apparatus does not need means for supply of special detergents, but in the second case this can be needed.

A known apparatus for the purpose has a treatment chamber with a lid and a drain through a water seal. The lid is operated by means of a foot switch which also serves as a switch for starting an automatic control system, with which a complete operation sequence is performed in the apparatus before the lid can be opened again. The apparatus is connected to one conduit for cold water, one for warm water and one for hot water. The treatment in this known apparatus is performed in the following way, a used bucket being taken as an example. The bucket is first emptied of its contents direct into the treatment chamber of the apparatus and thereafter placed therein according to a special instruction. Thereafter the process is started by the foot switch, the lid is closed and locked. From the connection points of the apparatus for water, conduits with valves go to spray nozzles in the wall of the treatment chamber. First a cold water flushing is made in order to dissolve certain types of contaminants and to wash away coarse impurities on the chamber wall. This flushing can be dispensed with if these types of contaminants or impurities are not present on the goods to be treated.

Thereafter a warm water flushing is made which raises the temperature of the goods. This results in that goods and treatment chamber are cleaned. Then flushing with hot water is made with the object of raising the temperature of the goods and keep it above 85°C during at least 40 sec. This flushing is intended to cause disinfection of the goods. Finally a short flushing with warm water is made in order to lower the temperature of the goods so that it will be easy to handle.

If hot water is not available, the apparatus can be provided with a water heater or connected to a steam conduit.

However, the apparatus has a plurality of conduits with several control valves. For one treatment a considerable quantity of water is consumed, and the hot water flushing requires a large quantity of energy.

Another apparatus for the same purpose is known from the French patent specification No. 1,565,407. It has a built in steam generator, which forms part of the water supply conduit to nozzles in a treatment chamber. Therefore it is more energy consuming than it should be.

The object of the present invention is to provide a method of disinfection which can be performed with a simple apparatus without control valves, with a limited water quantity and with a small consumption of energy.

For the said purpose the method according to the invention is mainly characterized therein that the goods are emptied of their contents and are placed in the treatment chamber which is closed, that a quantity of water is collected at a given temperature in a vessel that water is pumped from the vessel through a conduit and spray nozzles into the chamber and into the goods, that water is supplied from the conduit through an open branch conduit to the steam generator below the conduit interrupting said water supply, that water in the steam generator is heated to steam generation, that the steam thus formed of water present in the steam generator is conducted through the said conduit with the pump inactive, whereby the goods are heated above 85°C, whereafter water is supplied to the nozzles from the vessel by the pump.

An arrangement for performing the method is mainly characterized by a vessel for collecting a given quantity of tempered water, a conduit with a pump from the vessel to nozzles in the treatment chamber and by the steam generator being connected to the conduit in an open branch conduit downstream the pump and below the conduit.

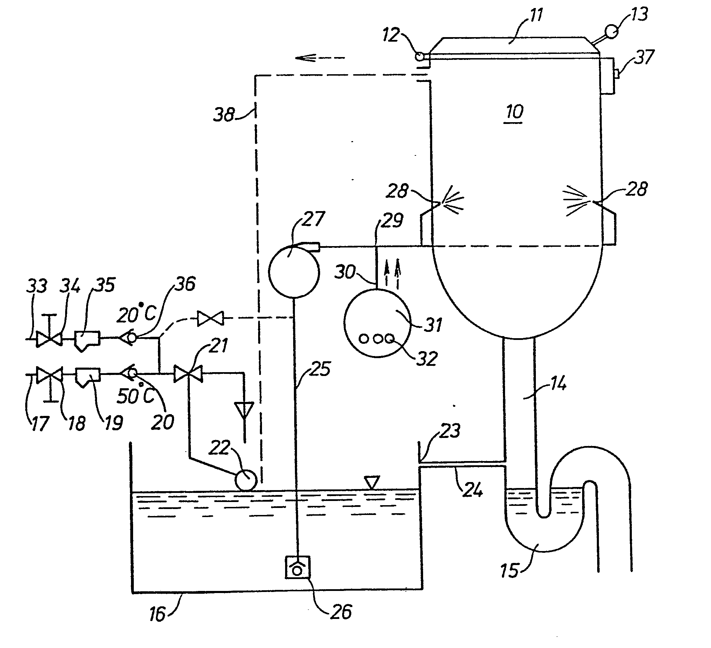

The invention will be described in the following with reference to a flusher disinfector chosen as an example and shown schematically in the drawing.

The flusher disinfector according to the invention has a treatment chamber 10 with a lid 11 having hinges 12 and a handle 13. From the bottom of the chamber 10 a drain conduit 14 goes through a water seal 15.

The apparatus further comprises a vessel 16 for water adapted to hold a quantity of water which is less than the quantity needed for a treatment. Water is supplied to the vessel 16 through a conduit 17 in which the water has a temperature of about 50°C. It is suitable to use water of a temperature between 40 and 60°C in the vessel 16. The conduit 17 has a shut-off valve 18, a water filter 19, a non-return valve 20 and a float valve 21 with a float 22. The vessel also has a spillway 23 connected to the drain conduit 14 from the chamber 10 through a conduit 24.

According to the invention a water conduit 25 is arranged from a point close to the bottom of the vessel 16 through a non-return valve 26 and a pump 27 to spray nozzles 28 in the chamber 10. Between the pump 27 and the chamber 10 the conduit 25 has an open branch connection in a point 29 through a conduit 30 to a steam generator 31 with electric heating elements 32.

Further there is a supply conduit 33 for cold water to the vessel 16. This conduit, like the warm water conduit 17, has a shut-off valve 34, a filter 35 and a non-return valve 36. The cold water conduit 33 opens into the conduit 17 between its non-return valve 20 and the float valve 21. Therefore it is possible to mix in water in the vessel 16 to a desired temperature. Between the float valve 21 and the two supply conduits 17, 33 a thermostat valve can be arranged which controls the temperature of the water supplied to the vessel 16. During operation of the pump 27 the float valve is acted upon and the vessel 16 is supplied with water.

A distinfection is made in the following way in the apparatus.

The vessel 16 is filled with water of suitable temperature. The goods are emptied of their contents into the treatment chamber 10 and are placed in a suitable way in relation to the nozzles 28. The lid 11 is closed. The process is started by a switch 37. Thereby the lid is locked and the pump 27 is started. Further a first timer is started. Also the steam generator 31, which is filled with water, is put in operation. After a given time, for instance 40 sec, the goods have been flushed clean. Then the activity of the pump is interrupted for abt. 10 sec waiting for the steam generator 31 to start delivery of steam. The steam is conducted to the branch conduit 30, the water conduit 25 and the nozzles 28 into the chamber 10, in which the temperature of the goods is raised. The time for the steam supply has to be so adjusted that the goods are above 85°C during at least 40 sec. The entire stream treatment can take 100 sec. The steam quantity needed can be obtained from a small quantity of water of a few litres, about $\frac{1}{2}$ litre of which being vapourized and conducted to the chamber 10.

After treatment of the goods at high temperature cooling is made by water spray. 5 sec are sufficient to lower the temperature of the goods below 50°C so that it will be easy to handle. A second timer disconnects the pump and opens the lock for the lid 11. At the same time it is indicated, for instance by a lamp which is extinguished or lighted, that the process is finished and that the goods can be taken out.

When the pump operates after the steam treatment the steam generator is filled with water and is prepared for the next treatment.

Several timers or a single one with several steps can be used controlling the process. The chamber 10 can be vented to a central ventilation arrangement for the treatment room. It can also be ventilated through a special conduit 38 which is shown by dashed lines to the water vessel 16. However, it seems to be best to use the conduit 24 from the spillway 23 for the purpose. In that case this conduit is disinfected in each treatment. It is suitable to have a lid for the vessel 16.

## Claims

1. Method of disinfecting objects used for the personal care of patients in a flusher disinfector with a chamber for the goods and a steam generator, characterized therein that the goods are emptied of their contents and are placed in the treatment chamber (10) which is closed, that a quantity of water is collected at a given temperature in a vessel (16), that water is pumped from the vessel (16) through a conduit (25) and spray nozzles (28) into the chamber (10) and into the goods, that water is supplied from the conduit (25) through an open branch conduit (30) to the steam generator (31) below the conduit (25), interrupting said water supply, that water in the steam generator is heated to steam generation, that the steam thus formed of water present in the steam generator is conducted through the said conduit (25) with the pump inactive, whereby the goods are heated above 85°C, whereafter water is supplied to the nozzles (28) from the vessel (16) by the pump (27).

2. Method according to Claim 1, characterized therein that heating of the steam generator (31) is started before pumping of water is interrupted.

3. Arrangement in a flusher disinfector with a chamber for the goods and a steam generator for performing the method according to Claim 1, characterized by a vessel (16) for collecting a given quantity of tempered water, a conduit (25) with a pump (27) from the vessel (16) to nozzles (28) in the treatment chamber (10) and by the steam generator (31) being connected to the conduit (25) in an open branch conduit (30) downstream the pump (27) and below the conduit (25).

4. Arrangement according to Claim 3, characterized therein that the conduit (25) has a non-return valve (26).

5. Arrangement according to Claim 3, characterized therein that a vent conduit (38) is arranged between the treatment chamber (10) and the vessel (16).

6. Arrangement according to Claim 5, characterized therein that the vessel (16) has a spillway (23) connected through a conduit (24) to a drain (14) from the treatment chamber (10) and that the spillway conduit (24) forms ventilation for the treatment chamber (10).

7. Arrangement according to Claim 3, characterized therein that the control means for the disinfector comprise members for time control of a switch for the motor of the pump (27) and for starting and stopping means for the steam generator (31).

## Patentansprüche

1. Verfahren zum Desinfizieren von für die persönliche Pflege von Patienten bestimmten Gegenständen in einem Spülsterilisator mit einer Kammer für die Gegenstände und einem Dampfgenerator, dadurch gekennzeichnet, daß die Gegenstände entleert und in der Behandlungskammer (10) angeordnet werden, die geschlossen wird, daß eine Wassermenge mit gegebener Temperatur in einem Behälter (16) gesammelt wird, daß Wasser aus dem Behälter (16) durch eine Leitung (25) und Sprühdüsen (28) in die Kammer (10) und in die Gegenstände gepumpt wird, daß Wasser von der Leitung (25) durch eine offene Zweigleitung (30) zum Dampfgenerator (31) unterhalb der Leitung (25) geleitet wird, daß diese Wasserzufuhr unterbrochen, Wasser im Dampfgenerator zwecks Dampfbildung erhitzt, der so aus dem im Dampfgenerator vorhandenen Wasser gebildete Dampf durch die Leitung (25) bei inaktiver Pumpe geleitet wird, sodaß die Gegenstände über 85°C erhitzt werden, worauf Wasser aus dem Behälter (16) den Düsen (28) durch die Pumpe (27) zugeführt wird.

2. Verfahren nach Anspruch 1, dadurch gekennzeichnet, daß das Erhitzen des Dampfgenerators (31) begonnen wird, bevor das Wasserpumpen unterbrochen wird.

3. Anordnung in einem Spülsterilisator mit einer Kammer für die Gegenstände und einem Dampfgenerator zur Durchführung des Verfahrens nach Anspruch 1, gekennzeichnet durch einen Behälter (16) für die Aufnahme einer gegebenen Menge von temperiertem Wasser, einer Leitung (25) mit Pumpe (27) vom Behälter (16) zu Düsen (28) in der Behandlungskammer (10) und durch einen Dampfgenerator (31), der an die Leitung (25) mit einer offenen Zweigleitung (30) stromabwärts der Pumpe (27) und unterhalb der Leitung (25) angeschlossen ist.

4. Anordnung nach Anspruch 3, dadurch gekennzeichnet, daß die Leitung (25) ein Rückschlagventil (26) aufweist.

5. Anordnung nach Anspruch 3, dadurch gekennzeichnet, daß zwischen der Behandlungskammer (10) und dem Behälter (16) eine Entlüftungsleitung (38) angeordnet ist.

6. Anordnung nach Anspruch 5, dadurch gekennzeichnet, daß der Behälter (16) einen Überlauf (23) besitzt, der durch eine Leitung (24) an einen Abfluß (14) von der Behandlungskammer (10) angeschlossen ist, und daß die Überlaufleitung (24) eine Belüftung für die Behandlungskammer (10) bildet.

7. Anordnung nach Anspruch 3, dadurch gekennzeichnet, daß die Steuerung für den Desinfektionsapparat Elemente für die zeitabhängige Steuerung eines Schalters für den Motor der Pumpe (27) und für die In- und Außerbetriebnahme des Dampfgenerators (31) aufweist.

## Revendications

1. Procédé de désinfection d'objets pour l'usage personnel de patients dans un stérilisateur à aspersion ayant une chambre pour les objets et un générateur de vapeur, caractérisé en ce que les objets sont vidés de leur contenu et sont placés dans la chambre de traitement (10) qui est fermée, en ce qu'une quantité d'eau est recueillie à température donnée dans un récipient (16), en ce que de l'eau est pompée, à travers un conduit (25) et des ajutages d'arrosage (28), depuis le récipient (16) dans la chambre (10) et dans les objets, en ce que de l'eau est fournie, à travers un conduit de dérivation ouverte (30), du conduit (25) au générateur de vapeur (31) situé au-dessous du conduit (25), en ce qu'on arrête ladite alimentation en eau, en ce que de l'eau se trouvant dans le générateur de vapeur est chauffée au point d'une génération de vapeur, en ce que la vapeur ainsi produite à partir de l'eau se trouvant dans le générateur de vapeur est conduite à travers ledit conduit (25), la pompe étant inactive, de sorte que les objets sont chauffés à une température supérieure à 85°C, et en ce qu'ensuite de l'eau est fournie aux ajutages (28) par la pompe (27) depuis le récipient (16).

2. Procédé selon la revendication 1, caractérisé en ce que le chauffage du générateur de vapeur (31) est commencé avant l'interruption de pompage de l'eau.

3. Dispositif dans un stérilisateur à aspersion ayant une chambre pour les objets et un générateur de vapeur, pour la mise en oeuvre du procédé selon la revendication 1, caractérisé par un récipient (16) pour recueillir une quantité donnée d'eau chauffée, par un conduit (25) ayant une pompe (27) et s'étendant depuis le récipient (16) jusqu'à des ajutages (28) dans la chambre de traitement (10), et par le fait que le générateur de vapeur (31) est relié au conduit (25) dans un conduit de dérivation ouverte (30) en aval de la pompe (27) et au-dessous du conduit (25).

4. Dispositif selon la revendication 3, caractérisé en ce que le conduit (25) comporte une soupape de retenue (26).

5. Dispositif selon la revendication 3, caractérisé en ce qu'un conduit d'aération (38) est disposé entre la chambre de traitement (10) et le récipient (16).

6. Dispositif selon la revendication 5, caractérisé en ce que le récipient (16) comporte un trop-plein (23) relié par un conduit (24) à un conduit de déchage (14) de la chambre de traitement (10), le conduit de trop-plein (24) formant une aération pour la chambre de traitement (10).

7. Dispositif selon la revendication 3, caractérisé en ce que les moyens de commande du stérilisateur comprennent des organes destinés à la commande à programme d'un interrupteur pour le moteur de la pompe (27) et de moyens de mise en marche et d'arrêt du générateur de vapeur (31).